# EUROPÄISCHE PATENTSCHRIFT

(11) **EP 1 307 258 B1**
(45) Veröffentlichungstag und Bekanntmachung des Hinweises auf die Patenterteilung: **30.04.2008**
(21) Anmeldenummer: 00956178.8
(22) Anmeldetag: 13.07.2000
(51) Int. Cl.: A61N 1/36, A61N 1/372

(54) **VORRICHTUNG FÜR DEN GESCHÜTZTEN BETRIEB VON NEUROPROTHESEN**
DEVICE FOR THE PROTECTED OPERATION OF NEUROPROSTHESES
DISPOSITIF PERMETTANT LE FONCTIONNEMENT PROTEGE DE NEUROPROTHESES

(30) Priorität: 23.12.1999 DE 19962915
(43) Veröffentlichungstag der Anmeldung: 07.05.2003
(73) Patentinhaber: IMI Intelligent Medical Implants AG, 6304 Zug (CH)
(72) Erfinder: ECKMILLER, Rolf, 41460 Neuss (DE); BECKER, Michael, 53115 Bonn (DE); HÜNERMANN, Ralph, 50931 Köln (DE); ORTMANN, Valerij, 53757 Sankt Augustin (DE)
(74) Vertreter: Graf von Stosch, Andreas
(86) Internationale Anmeldenummer: PCT/EP2000/006666
(87) Internationale Veröffentlichungsnummer: WO 2001/047598

(56) Entgegenhaltungen:
- WO-A-86/02567
- US-A- 5 169 384
- US-A- 5 752 976
- US-A- 5 954 758

## Beschreibung

Die Erfindung betrifft eine Neuroprothese mit den Merkmalen des Oberbegriffs des Anspruchs 1.

Unter Neuroprothesen sind im Zusammenhang mit der vorliegenden Patentanmeldung Vorrichtungen zum Einsatz in Kontakt oder Wirkungszusammenhang (unidirektionale oder bidirektionale Beeinflussung durch Ausschüttung von Wirkstoffen) mit dem zentralen Nervensystem innerhalb des Schädels, des Rückenmarks oder mit dem Rückenmark verbundener peripherer Nerven, sowie Sehprothesen oder Hörprothesen mit einer implantierten internen Komponente und einer nicht implantierten externen Komponente zu verstehen.

Es sind mehrere Neuroprothesen mit einer implantierten Komponente in Kontakt mit Nervengewebe u.a. zur Behandlung von Funktionsstörungen des Sehsystems, des Hörsystems, des intrakraniellen Nervensystems, des vegetativen Nervensystems, des Rückenmarks, oder des peripheren Nervensystems bekannt, bei denen eine Datenübertragung zwischen einer externen und einer internen, implantierten Komponente zum Zwecke des Betriebes, der Funktionsüberwachung, oder der Funktionsfestlegung vorgesehen ist, z.B. aus den WO 98/36793, WO 98/36795 und US 6,002,966 .

Es sind mehrere Systeme zur geschützten Datenübertragung zwischen voneinander entfernten Komponenten u.a. im Mobilfunk, der Satelliten-Kommunikation, oder bei lokalen Computernetzen oder dem Internet z.B. für Tele-Banking, PKW Diebstahlsicherung oder Tele-Chirurgie bekannt, z.B. aus den Druckschriften DE 2618401, US 5,646,456, US 5,734,330, US 5,940,515, US 5,930,362, US 5,963,621, US 5,940,799, DE 19630920 und EP 0946018

Es sind Implantate ohne Kontakt zu Nervengewebe bekannt, die u.a. zur Personenidentifikation oder zur Standortverfolgung von Tieren in Signal-Verbindung mit einer externen Komponente stehen, z.B. aus US4,399,821, US 4,909,250, US 5,855,609, WO 97/00708, EP 0896 828 A2 und WO 98/29160

Es sind Implantate als Herzschrittmacher bekannt, deren Betriebszustand über eine externe Komponente überprüft oder/und verändert werden kann, z.B. aus US 4,361,153, DE 2944542, US 5,871,451 und US 5,891,178

Es gibt Verschlüsselungs- und Entschlüsselungs-Vorrichtungen und -Verfahren zur Vermeidung des nicht autorisierten Zugriffs auf digitale oder analoge Datenübertragungen u.a. in technischen oder medizintechnischen Anwendungsgebieten, z.B. aus US 4,766,516, US 5,365,225, US 5,696,825, US 5,987,440 und WO 98/10836

Die Internationale Patentanmeldung WO 86/02567 offenbart ein Verfahren und Gerät zur elektrischen Stimulation von Nervengewebe. Die Applizierten elektrischen Signale sollen einen therapeutischen Effekt haben und beispielsweise Schmerzen lindern. Eine Nervenstimulationsvorrichtung bestehend aus einem Personal Delivery Instrument, einer Steuereinheit und einer Entwicklungsstation 20 ist vorgesehen. Das Personal Delivery Instrument ist über eine Leitung 22 mit dem Patientenkopf 21 verbunden. Ein Implantat für den Patienten ist vorgesehen, zu dem eine Funkübertragung besteht.

Die gegenwärtig konzipierten bzw. verfügbaren Neuroprothesen oder Neuroimplantate, wie z.B.: Cochlear Implant für Gehörlose, lernfähiges Retina Implant für Blinde mit Netzhautdegeneration haben keine Zugangskontrolle, die den Zugriff auf Daten und Betrieb von einer spezifischen Autorisierung abhängig macht. Deswegen können im Prinzip bei einem gegebenen Implantatträger die für ihn speziell eingestellten externen Komponenten ausgetauscht bzw. verwechselt werden und so zu erheblichen und ggf. schädlichen Funktionsstörungen im technischen oder/und biomedizinischen Bereich führen.

Ferner können schutzbedürftige personenbezogene Daten oder/und implantatbezogene Daten zum Schaden des Implantatträgers oder/und des Implantatherstellers/Betreibers unautorisiert abgerufen oder/und verändert werden.

Stattdessen wird gegenwärtig für den Neuroprothesen-Betrieb typisch eine Kommunikationsverbindung zwischen der fest mit dem Implantatträger verbundenen implantierten Komponente und einer prinzipiell austauschbaren externen Komponente aufgebaut ohne eine hinreichende Vorsorge zum Schutz vor dem unautorisierten Datenzugriff usw. getroffen zu haben. Daher ist das prinzipielle Risiko eines Missbrauchs bzw. eines nicht beabsichtigten Fehlbetriebes nennenswert.

Es ist deshalb die Aufgabe der vorliegenden Erfindung, diese Nachteile zu beseitigen und ein Neuroimplantat-Schutzsystem (NIS) zu offenbaren, dass den unautorisierten Zugriff verhindert.

Weil der Datentransfer bzw. der Betrieb der Neuroprothese nur nach einer Autorisierung mithilfe der implantierten Komponente erfolgen kann, werden diverse Formen eines denkbaren Betriebsmissbrauches verhindert.

Die bevorzugten Ausgestaltungsformen des Autorisierungsvorganges stützen sich auf wesentliche Funktionsmerkmale der implantierten Komponente, die im implantierten Zustand nicht ausgeforscht, kopiert oder simuliert werden können.

Da der Autorisierungsvorgang das Zusammenwirken der implantierten mit der externen Komponente zwingend voraussetzt, können geschützt gespeicherte Daten in der internen, der externen und gegebenenfalls einer weiteren internen Komponente nicht nur Daten für Zwecke der Neuroprothese, sondern auch z.B. wirtschaftlich oder juristisch wichtige personenbezogenen Daten einschließen.

Aufbau und Autorisierungssystem einer so ausgestalteten Neuroprothese verhindern den Nachbau der einzelnen Komponenten.

Das hier offenbarte Neuroimplantat-Schutzsystem (NIS) verschafft den so erweiterten bzw. zusätzlich ausgestatteten Neuroprothesen eine Reihe von wesentlichen Vorteilen gegenüber bisherigen Neuroprothesen ohne NIS. Damit wird die Betriebssicherheit und die Akzeptanz von Neuroprothesen u.a. in Bezug auf Datenschutz und Schutz vor Bedienungsfehlern wesentlich erhöht.

Erstmals wird hier ein System offenbart, dass Betriebsmissbrauch durch Kopplung der implantierten Komponente mit nicht speziell zugeordneten externen Komponenten verhindert. Damit wird erstmals sichergestellt, dass der Implantatträger vor Funktionsschäden aufgrund des falschen Komponenten-Einsatzes geschützt wird.

Durch diese Erfindung wird es für mit NIS ausgestattete Neuroprothesen erstmals möglich, den nicht autorisierten Zugang zu den Daten und Funktionsfestlegungen der implantierten Komponente zu verhindern.

Ferner wird es durch diese Erfindung erstmals möglich, den nicht autorisierten Zugang zur Datenübertragung zwischen externer und implantierter Komponente zu verhindern. Dadurch wird insbesondere der personenbezogene Datenschutz des Implantatträgers, der bei herkömmlichen Heilverfahren u.a. durch die Schweigepflicht des behandelnden Arztes geschützt wird, bezüglich des Neuroprothesen-Betriebes erstmals nachhaltig gesichert.

Zusätzlich kann durch die offenbarte Erfindung der nicht autorisierte Zugang zu wesentlichen Funktionseigenschaften der Neuroprothese verhindert werden. Damit wird der nicht autorisierte Nachbau (Reverse Engineering) von Komponenten der so ausgestatteten Neuroprothesen verhindert, da einerseits wesentliche Funktionseigenschaften für den nicht autorisierten Nachbau fehlen und da andererseits derartige nicht autorisierte Nachbaukomponenten nicht mit den übrigen Komponenten wegen fehlender Kompatibilität und fehlender Autorisierung der Komponentenkommunikation betrieben werden können.

Im folgenden werden Ausführungsbeispiele von vorteilhaften Ausgestaltungen des Neuroimplantat-Schutzsystems (NIS) und der zugehörigen Verfahren anhand der Zeichnung dargestellt. Es zeigen:
Figur 1: ein Schema des Neuroimplantat-Schutzsystems (NIS) für eine Neuroprothese;
Figur 2: das Schema eines bei der Fertigung zugeordneten Paares von externer (Schlüssel) und interner (Schloss) Komponente
Figur 3: das Schema einer bevorzugten Realisierung der mikroelektronischen Festlegung der Schlüssel / Schlossfunktion in der implantierten internen Komponente bzw. in der externen Komponente
Figur 4: das Schema einer bevorzugten Ausführung eines 'Dynamischen Labyrinthes' zur Repräsentation des zweiten Autorisierungssignals als Schloss in der internen Komponente; sowie
Figur 5: eine weitere bevorzugte Ausgestaltung für das Schema des Neuroimplantat-Schutzsystems (NIS).

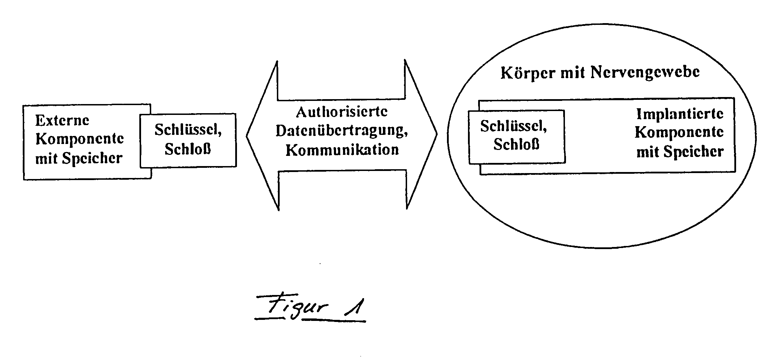

Fig. 1 zeigt ein Schema des Neuroimplantat-Schutzsystems (NIS) für eine Neuroprothese. Sowohl die externe Komponente der Neuroprothese, als auch die implantierte, interne Komponente verfügen über einen Schlüssel oder / und ein Schloss für die Prüfung und Durchführung von autorisierten Datenübertragungen oder / und zu Abfragen oder / und Neufestlegungen des Funktionszustandes der einzelnen Komponenten.

Der Betrieb der Neuroprothese bzw. der autorisierte Zugriff auf intern oder extern gespeicherte Daten ist nur möglich, wenn ein von der externen Komponente bei der internen Komponente eintreffendes Autorisierungssignal nach einem Verfahren legitimiert worden ist, welches allein auf Merkmalen der internen Komponente basiert und nicht von einer externen Komponente aus analysiert, verändert, oder umgangen werden kann.

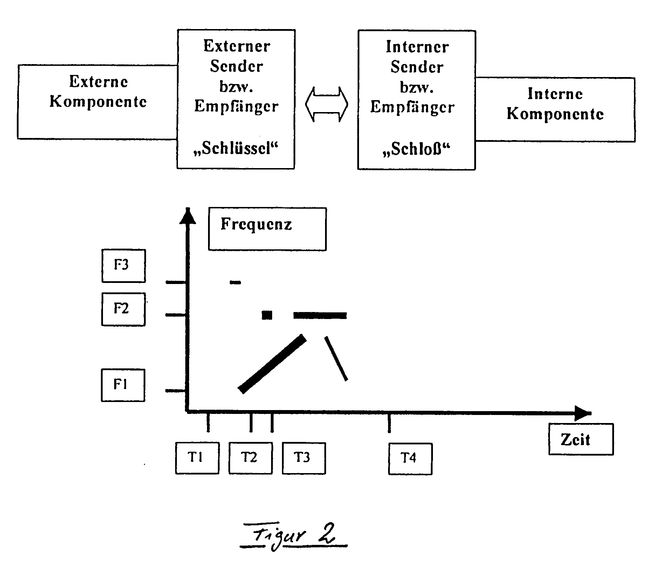

Fig.2 zeigt das Schema eines bei der Fertigung zugeordneten Paares von externer (Schlüssel) und interner (Schloss) Komponente ohne die Möglichkeit der Entdeckung des Schlüssels oder des Schlosses durch Analyse der externen Komponente. In einer hier skizzierten bevorzugten Ausgestaltung wird angenommen, dass aus einem größeren Frequenzbereich (Schall, elektromagnetische Wellen, Licht, Rauschgenerator, usw.) für das in dem Frequenz-Zeit-Diagramm als einfaches Beispiel skizzierte Autorisierungs-Signal die Frequenzen F1, F2, F3 sowie die Zeitfolge T1, T2, T3, T4 während der Paar-Fertigung ausgewählt wurden. Ferner wurde das skizzierte Frequenz-Zeit-Muster zur Autorisierung bzw. Identifikation für dieses Paar ausgewählt. Die zugehörigen Parameter- und Funktionsfestlegungen in der externen und in der internen Komponente erfolgten während bzw. nach der Fertigung zum Teil durch Festlegungen in der zugehörigen Mikroelektronik bzw. Mikromechanik und zum Teil durch Festlegungen in der Software.

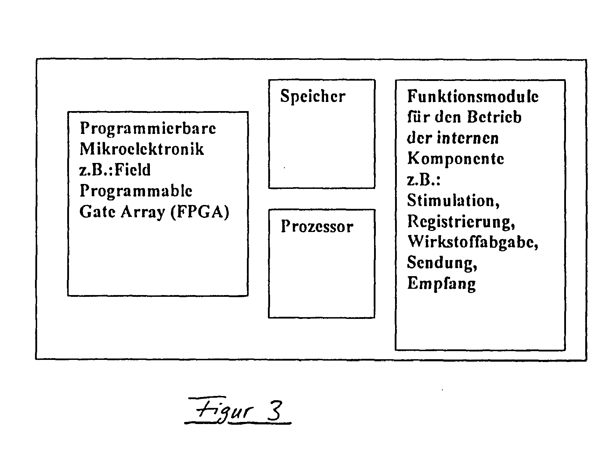

Fig.3 zeigt das Schema einer bevorzugten Realisierung der mikroelektronischen Festlegung der Schlüssel / Schlossfunktion in der implantierten internen Komponente bzw. in der externen Komponente. Das ausgewählte Autorisierungssignal ist z.B. ein Signal-Muster, welches durch Amplituden-, Frequenz-, Zeit- und Orts-Parameter (im Prinzip z.B. vergleichbar der Vokalisation eines Tieres) festgelegt ist. Zum Zweck der Autorisierung wird in einer bevorzugten Ausführung durch das Zusammenwirken von Speicher, Prozessor und FPGA o.ä. während oder nach der Fertigung eine Art Labyrinth erzeugt, welches das Schloß repräsentiert und welches nur vom einzig richtigen Autorisierungssignal als Schlüssel durchlaufen werden kann. Das richtige Durchlaufen des 'Labyrinthes' erzeugt seinerseits in einer bevorzugten Ausführung ein zeitliches Signalmuster, welches in einer mikroelektronisch festgelegten Form auf eine logische Gatterstruktur geleitet und als "Ja=Autorisierung kann stattfinden" interpretiert wird. Weder die Struktur und Funktion des Labyrinthes, noch die längs des Labyrinthes angekoppelte Gatterfunktion oder Struktur, die vollständig der implantierten, internen Komponente zugeordnet sind, können durch Einwirkungen seitens der externen Komponente transparent gemacht, kopiert oder simuliert werden.

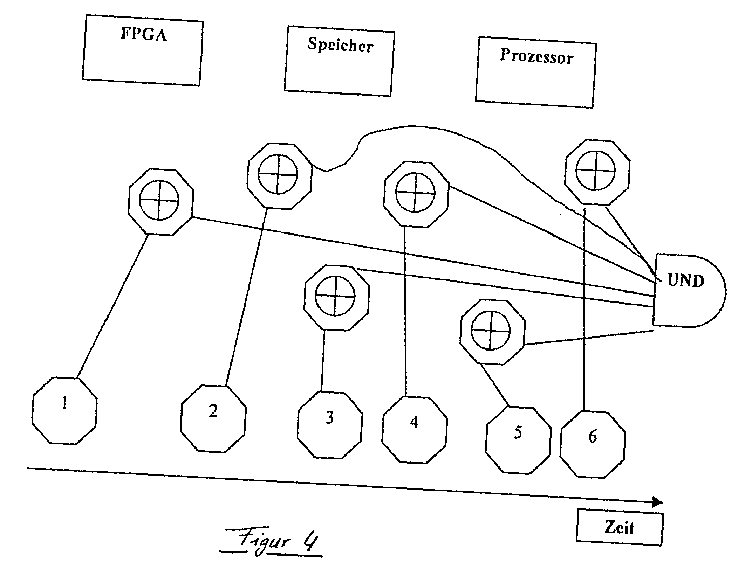

Fig. 4 zeigt das Schema einer bevorzugten Ausführung eines ,Dynamischen Labyrinthes' zur Repräsentation des zweiten Autorisierungssignals als Schloss in der internen Komponente. Das ,Dynamische Labyrinth' (zum kleinen Teil angedeutet unten in Fig. 4 durch eine Sequenz von Instruktionen bzw. digitalen Zuständen, von denen z.B. sechs als Koppelpunkte 1 bis 6 laufend beobachtet werden) kann vorzugsweise durch einen Algorithmus repräsentiert sein, der durch ein festgelegtes Zusammenwirken von programmierbarer Mikroelektronik (z.B. FPGA), Speicher oder/und Prozessor realisiert wird und der die einzelnen Elemente eines eintreffenden Signales jeweils logischen Funktionen und Informationsverarbeitungspfaden zuordnet, so dass sich das Signal als Funktion der Zeit über dieses Labyrinth verteilt bzw. schrittweise in ihm fortschreitet. In einer bevorzugten Ausgestaltung ist vorgesehen, dass nur, wenn das von der externen Komponente empfangene Signal als Autorisierungssignal und somit als Schlüssel aufgrund der in programmierbare Hardware, Speicher und Prozessor der internen Komponente die Koppelpunkte 1 bis 6 zu den vorgegebenen Zeiten mit dem jeweils für die Autorisierung vereinbarten Wert erreicht und hier z.B. von einem Schaltnetz oder Schaltwerk (Oktagone mit Kreuz und Kreis als angedeutete logische Bausteine einschließlich Zeitverzögerungsglieder mit den angedeuteten Verbindungsleitungen) detektiert wird und die Ausgangssignale des Schaltnetzes wiederum von dem rechts angedeuteten UND-Gatter in einem engen Zeitfenster als logische Eins bewertet werden, die Autorisierung vollzogen wird. Die zugehörigen Zeitfestlegungen und logischen sowie Labyrinth-Festlegungen sind in dieser bevorzugten Ausgestaltung nicht explizit als Algorithmen verfügbar, sondern sind in die interne Komponente eingebettete Festlegungen, die nicht ausgeforscht werden können und wegen der Implantation nicht zugänglich sind. Erst wenn die Autorisierung erfolgt ist, können Neuroprothesen-Betrieb, Datenübertragung oder / und Zugriff auf geschützte Speicherbereiche der einzelnen Komponenten erfolgen. Die Autorisierung kann während des Betriebes erneuert werden, wobei in einer bevorzugten Ausgestaltung nicht das zur primären Autorisierung verwendete Autorisierungssignal wiederholt verwendet wird, sondern hierfür ein neues Signal verwendet wird.

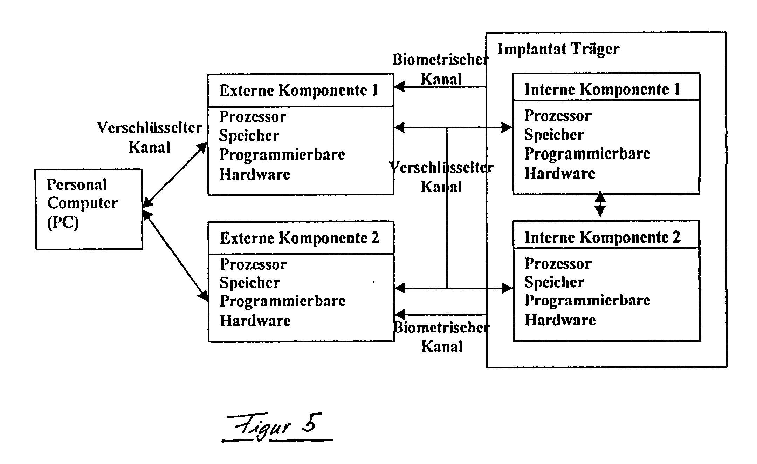

Fig. 5. zeigt eine weitere bevorzugte Ausgestaltung für das Schema des Neuroimplantat-Schutzsystems (NIS). Ein NIS besteht aus z.B. zwei implantierten Komponenten und geschützten Datenübertragungskanälen, zur Kommunikation zwischen implantierten Komponenten sowie zwischen internen Komponenten und z.B. zwei externen Komponenten und/oder einem PC sowie z.B. zur Übertragung biometrischer Daten vom Implantatträger an eine der externen Komponenten oder/und einen PC. Die Kommunikation findet verschlüsselt statt. Externe und interne Komponente beinhalten dazu notwendige Verschlüsselungshardware und/oder -Software auf der Basis eines Prozessors und/oder programmierbarer Hardware und/oder von Speichern. Der Betrieb erfolgt nur nach einer erfolgreicher Authentifizierung und Autorisierung aller Komponenten.

Eine vorteilhafte Ausgestaltung des Verfahrens zum geschützten Betrieb einer Neuroprothese (s. Fig. 1, Fig.2, Fig. 3, Fig. 4, Fig. 5) besteht darin, dass die externe Komponente ein verschlüsseltes Autorisierungssignal durch ein gängiges elektromagnetisches (z.B. als frequenzmodulierte Pulsgruppe), oder/und optoelektronisches Verfahren (z.B. als amplitudenmodulierte Pulsgruppe von einer Laser-Diode als Sender zu einem Photosensor als direkt unter der Haut oder innerhalb des Auges implantierter Empfänger), oder nach anderen physikalischen oder chemischen Prinzipien wie z.B. die Modulation von Schall einschließlich Körperschall mit technisch bekannten Sendern und Empfängern, oder die spezifische Applikation eines mechanischen Signals (z.B. die Erzeugung von Vibrationsmustern mit einem oder mehreren, örtlich verteilten sehr lokal wirkenden Vibratoren in Kommunikation mit entsprechenden technisch bekannten Vibrationssensoren) mit einer hierfür speziell ausgestalteten und / oder positionierten Teilstruktur der implantierten, internen Komponente zum Zweck der für den Neuroimplantat-Betrieb oder/und für die Datenübertragung zwischen externer und interner Komponente erforderlichen Autorisierung austauscht.

Eine weitere vorteilhafte Ausgestaltung des Verfahrens zum geschützten Betrieb besteht darin, dass die interne, implantierte Komponente neben einem periodischen über einen nicht-periodischen Zeitgeber verfügt, der die Zeit nach einem festgelegten Code einteilt und zum Zwecke der Autorisierung eine Kopie dieses Zeitcode-Gebers in der externen Komponente benötigt. Zu diesem Zweck wird z.B. während der Fertigung bzw. Justierung eines Paares von externer und implantierter, interner Komponente in beiden Komponenten ein Algorithmus zur Erzeugung von zueinander identischen, nicht-periodischen Zeitreihen festgelegt. Ferner wird in dieser bevorzugten Ausgestaltung z.B. durch vereinbarte Synchronisiersignale der Gleichlauf beider nicht-periodischer Zeitgeber sichergestellt. Damit können u.a. nicht-autorisierte Zugangsversuche zu Daten oder Betrieb der Neuroprothese, die typischerweise auf periodischen Zeitgebern basieren, sehr schnell erkannt und abgewehrt werden.

Eine weitere vorteilhafte Ausgestaltung des Verfahrens zum geschützten Betrieb besteht darin, dass die interne Komponente, je nach Festlegung, im Falle der Ablehnung des Autorisierungsversuches entweder ein Ablehnungssignal geben, oder sich passiv verhalten und so seine Existenz und ggf. genaue Lokalisation geheim halten kann.

Eine weitere vorteilhafte Ausgestaltung des Verfahrens zum geschützten Betrieb besteht darin, dass je nach Festlegung in der Neuroprothese das Akzeptieren einer Autorisierung sehr unterschiedlich umgesetzt werden kann durch: a) automatischer Betriebs- oder Datenübertragungsbeginn ohne separates Akzeptanz-Signal, b) Erzeugung eines von der internen Komponente gesendeten Signales, welches auch ohne Zugang zum entsprechenden Code von entsprechenden extern positionierten Sensoren detektiert werden kann und c) Verstellung einer passiven Eigenschaft der internen Komponente (z.B. das Einschalten eines internen Energieempfängers, Schwingkreises, oder von Absorptions- oder Reflexionsstrukturen), die zwar von der speziell autorisierten Komponente, die z.B. im autorisierten Code sendet, extern z.B. durch erhöhten Energieabfluss messbar ist, jedoch nicht von fremden externen Detektoren entdeckt werden kann.

Eine weitere vorteilhafte Ausgestaltung des Verfahrens zum geschützten Betrieb besteht darin, dass zur Vermeidung von Betriebsmissbrauch grundsätzlich eine interne Komponente nur genau einer externen Komponente zugeordnet ist und umgekehrt. Diese eindeutige Exklusiv-Zuordnung ist so realisiert, dass sie nicht durch in der Datenverarbeitung gängige Zugangskontrollmechanismen mit "Super user" Rechten z.B. des Implantat-Betreibers oder Herstellers oder durch Kenntnis eines Passwortes außer Kraft gesetzt werden kann, sondern dass z.B. der Austausch einer ursprünglich der internen Komponente zugeordneten speziellen externen Komponente nur durch die ausdrückliche Zustimmung des Implantat-Trägers nach Art der Zustimmung zu einem medizinischen Eingriff erfolgen kann. Zu diesem vorteilhaften Zweck kann ein funktionelles Echtzeit-Modell der speziellen externen Komponente nur während des autorisierten Betriebes hergestellt bzw. während der Paarfertigung oder initialen Justierung eines Paares aus externer und interner Komponente ein Duplikat der externen Komponente hergestellt werden, das so gesichert verwahrt wird, dass nur der Implantatträger über den Zugang zu dieser "Kopie" als Modell bzw. Duplikat des für die Autorisierung und den Datenaustausch nötigen Moduls der externen Komponente verfügen kann.

Eine weitere vorteilhafte Ausgestaltung des Verfahrens zum geschützten Betrieb besteht darin, dass die Erlangung des autorisierten Zuganges zu Datenübertragung oder/und interner Komponente durch eine Funktionsanalyse der speziellen externen Komponente, oder dem ihr zugeordneten funktionellen Echtzeit-Modell außerhalb des autorisierten Betriebes nicht möglich ist. Diese vorteilhafte Eigenschaft wird u.a. dadurch realisiert, dass die in der externen Komponente während des Analysebetriebes erfassbaren Eigenschaften keinen hinreichenden Aufschluss über die zur erfolgreichen Autorisierung benötigte Codierung bzw. Verschlüsselung und Decodierung bzw. Entschlüsselung des Autorisierungssignals geben. Zu diesem Zweck wird die Autorisierung bevorzugt derart gestaltet, dass ein mehrdimensionales "Template Match", welches z.B. die Dimensionen: Zeit, Amplitude, Frequenz und Ort umfasst, und welches bei der paarweisen Fertigung von je einer internen und der zugehörigen externen Komponente in einem Zufallsprozess, oder mithilfe neuronaler Netze bzw. anderer Lernalgorithmen, oder unter Verwendung von nur dem Implantatträger eigenen Merkmalen wie z.B. : Iris des Auges, Fingerabdruck, Erbmaterial festgelegt wurde, ein unverwechselbares "Schlüssel-Schloss" Paar bildet. Dabei kann bei einer Funktions- und Strukturanalyse der externen Komponente weder der externe "Schlüssel", noch das zugehörige interne "Schloss" mit vernünftigem Aufwand ermittelt werden. Die bei dem Bemühen um unautorisierten Zugang denkbaren Tests, ob eine gerade gewählte Schlüssel-Kombination vom in der internen Komponente repräsentierten Schloss akzeptiert wird, sind bezüglich der Kombinationsmöglichkeiten derart groß, dass sehr schnell die vorgegebene Zahl vergeblicher Autorisierungsversuche erreicht und so die interne Komponente auf Dauer sperren würde.

Eine weitere vorteilhafte Ausgestaltung des Verfahrens zum geschützten Betrieb besteht darin, dass zur Herstellung bzw. zur initialen Justierung oder Funktionsfestlegung eines eindeutigen Paares von externer und interner Komponente sowie ggf. eines sicher zu verwahrenden Duplikates der externen Komponente bezüglich der Codierungs-Elemente jeweils Paare der zugehörigen Speicher oder/und Prozessor oder/und FPGA Bausteine in ihrer Funktion durch identische Software oder/und Hardware-Festlegungen behandelt werden. Z.B. kann das entsprechende Paar von programmierbaren Mikroelektronik-Bausteinen (z.B. FPGA) unter Berücksichtigung seiner physikalischen und geometrischen Eigenschaften durch einen identischen Prozess programmiert bzw. durch maschinelle Fertigungsschritte hergestellt werden, wodurch z.B. der nicht-periodische Zeitgeber oder die Festlegung der nur für dieses Paar verwendeten Familie von Autorisierungssignalen eindeutig, vertraulich und geschützt, nämlich eingebettet in die Mikroelektronik festgelegt wird. Diese identischen Funktionsfestlegungen bewirken aufgrund der in der externen und der internen Komponente verschieden festgelegten Funktionsabläufe und zusätzlicher Funktionen jedoch nicht identische Gesamtfunktionen, sondern genau eindeutig zu einander passende, komplementäre Funktionen. Dabei kann es sich um vom Hersteller wiederholbare oder nicht wiederholbare Funktionsfestlegungen handeln. Im Ergebnis entstehen so Paare, die nur miteinander, jedoch nicht mit anderen Komponenten kommunizieren bzw. Signale austauschen können.

Eine weitere vorteilhafte Ausgestaltung des Verfahrens zum geschützten Betrieb besteht darin, dass der Zugriff auf geschützte Daten oder Funktionszuständen sowohl in der internen Komponente, als auch in der externen Komponente von der erfolgreichen Autorisierung abhängig gemacht wird bzw. werden kann. Zu diesem Zweck wird in einer bevorzugten Ausführung ein in der internen Komponente legitimierter Autorisierungsversuch auch in der externen Komponente registriert und dort nicht nur zur Freischaltung der Datenübertragung, sondern auch zur Freischaltung des Zuganges zu in der externen Komponente geschützt verfügbaren Daten oder Funktionszuständen verwendet.

Eine weitere vorteilhafte Ausgestaltung des Verfahrens zum geschützten Betrieb besteht darin, dass das ausgewählte Autorisierungssignal z.B. ein Signal-Muster ist, welches durch Amplituden-, Frequenz-, Zeit- und Orts-Parameter festgelegt ist. Jede externe Komponente hat als Autorisierungssignal seine eindeutige Kennung als Autorisierungs-Schlüssel. Jede interne Komponente hat ihrerseits ein anderes Autorisierungssignal als Autorisierungs-Schloss. Kenntnis des Schlüssels oder des Schlosses allein erlaubt keinen Rückschluss auf das jeweils zur Autorisierung benötigte andere Signal. Nur, wenn in der internen Komponente das einzig richtige Autorisierungssignal als Schlüssel empfangen wird und unter Verwendung eines Speichers, oder/und eines Prozessors oder/und eines FPGA erfolgreich mit dem dort als Schloss verfügbaren Autorisierungssignal verglichen wird, so dass Schlüssel und Schloss gemeinsam zur Autorisierungsentscheidung führen, können Datenübertragung oder/und Neuroprothesenbetrieb gestartet werden.

Zum obengenannten Zweck wird in einer bevorzugten Ausführung durch das eventuelle Zusammenwirken von Speicher, Prozessor und programmierbare Hardware (s. Fig. 3) während oder nach der Fertigung eine Art 'Dynamisches Labyrinth' (Fig. 4) erzeugt, welches das Autorisierungssignal der internen Komponente das Schloss repräsentiert und welches nur vom einzig richtigen Autorisierungssignal als Schlüssel durchlaufen werden kann. Das richtige Durchlaufen des 'Labyrinthes' erzeugt seinerseits in einer bevorzugten Ausführung ein zeitliches Signalmuster, welches in einer mikroelektronisch festgelegten Form auf eine logische Gatterstruktur geleitet und als "Ja = Autorisierung kann stattfinden" interpretiert wird. Weder die Struktur und Funktion des Labyrinthes, noch die längs des Labyrinthes angekoppelte Gatterfunktion oder Struktur, die vollständig der implantierten, internen Komponente zugeordnet sind, können durch Einwirkungen seitens der externen Komponente transparent gemacht, kopiert oder simuliert werden.

Eine weitere vorteilhafte Ausgestaltung des Verfahrens zum geschützten Betrieb besteht darin, dass das 'Dynamische Labyrinth', vorzugsweise durch eine Sequenz von Instruktionen bzw. digitalen Zuständen, von denen einige als Koppelpunkte laufend beobachtet werden(s. Fig. 4) vorzugsweise durch einen Algorithmus repräsentiert ist, der durch ein festgelegtes Zusammenwirken von programmierbarer Hardware, Speicher oder/und Prozessor realisiert wird. Dieser bevorzugte Labyrinth-Algorithmus ordnet die einzelnen Elemente eines eintreffenden Signals jeweils logischen Funktionen und Informationsverarbeitungspfaden zu so dass sich das Signal als Funktion der Zeit über dieses Labyrinth verteilt bzw. schrittweise in ihm fortschreitet.

In einer bevorzugten Ausgestaltung des Labyrinthes besteht das Labyrinth, welches als Algorithmus nach dem Stand der Technik z.B. in FPGA, Speicher und/oder Prozessor implementiert werden kann, funktionell aus einer Zahl von Pfaden mit Gabelungen, Richtungsfestlegungen und Toren, die sich als Funktion der Zeit ändern. Ein typischer Versuch eines Signals, das Labyrinth erfolgreich zu durchlaufen, umfasst die Gliederung des Signals in einzelne Signalelemente, die an unterschiedlichen Stellen im Labyrinth gestartet werden und deren Zeitverlauf genau zum Zeitverlauf des jeweiligen Labyrinth-Pfades passen muß. Nur wenn sich also die Signalelemente genau passend zum Labyrinthpfad z.B. bezüglich Geschwindigkeit und Bewegungsrichtung ändern, können an festgelegten Koppelpunkten die geforderten Prüfsignale zu den geforderten Zeiten auftreten. In dieser bevorzugten Ausführung gilt, dass nur, wenn das von der externen Komponente empfangene Signal als Autorisierungssignal und somit als Schlüssel aufgrund des speziell festgelegten Labyrinth-Durchlaufes die Koppelpunkte zu den vorgegebenen Zeiten mit dem jeweils für die Autorisierung vereinbarten Wert erreicht und hier z.B. von einem Schaltnetz oder Schaltwerk (vorzugsweise bestehend aus logischen Bausteinen bzw. Funktionen einschließlich Zeitverzögerungsglieder und Verbindungsleitungen zu den einzelnen Koppelpunkten) detektiert wird und die Ausgangsignale der einzelnen Schaltnetzelemente (s. Fig. 4) wiederum von dem rechts angedeuteten UND-Gatter zeitlich koinzident bzw. in einem engen Zeitfenster als logische Eins bewertet werden, wird die Autorisierung vollzogen. Dabei wird die Koinzidenz der zu verschiedenen Zeiten an den Koppelpunkten detektierten Ereignisse durch Zeitverzögerungsglieder realisiert. Die zugehörigen Zeitfestlegungen und logischen Festlegungen des Schaltnetzes als Prüfsystem für die Zugehörigkeit von Schlüssel und Schloß sowie die das Schloß repräsentierenden Labyrinth-Festlegungen sind in die interne Komponente eingebettet und können daher nicht ausgeforscht werden und sind wegen der Implantation nicht zugänglich.

In einer bevorzugten Ausgestaltung des Verfahrens zum geschützten Betrieb wird der Vollzug der Autorisierung zur Verhinderung einer nicht autorisierten Vollzugsmeldung an die externe Komponente (Falschmeldung) nicht explizit an die externe Komponente gemeldet, sondern nur implizit durch die von der externen Komponente detektierbare Betriebsaufnahme der internen Komponente realisiert. Alternativ dazu wird der Vollzug jeweils durch ein nur einmal benutzbares und in der externen Komponente zwar richtig interpretierbares aber nicht explizit gespeichertes Signal von der internen an die externe Komponente gemeldet.

Eine weitere vorteilhafte Ausgestaltung des Verfahrens zum geschützten Betrieb besteht darin, dass die Autorisierung während des Betriebes verändert wird. Dies geschieht vorzugsweise dadurch, dass bereits bei der Festlegung von Autorisierungs-Schlüssel und Schloss im Zusammenhang mit der Fertigung eines Paares von externer und interner Komponente, der Wechsel des Schlüssels oder/und des Schlosses z.B. nach jeder erfolgreichen Autorisierung in der externen bzw. der internen Komponente vorbereitet wird und entsprechend automatisch erfolgt.

Eine weitere vorteilhafte Ausgestaltung des Verfahrens zum geschützten Betrieb besteht darin, dass in einer implantierten Komponente gespeicherte personenbezogene Daten, die grundsätzlich der ärztlichen Schweigepflicht unterliegen, wie z.B. Art und intendierter Wirkmechanismus der Neuroprothese, Quantität, Art oder Zeitverlauf der therapeutischen oder / und diagnostischen Maßnahme mit einer hierfür gesonderten Zugangssicherung geschützt sind.

Eine weitere vorteilhafte Ausgestaltung des Verfahrens zum geschützten Betrieb besteht darin, dass im Falle einer Betriebsstörung oder eines anders gelagerten Notfalles die interne Komponente abgeschaltet bzw. funktionell stillgelegt, oder auf ein vorbereitetes Notfallprogramm umgeschaltet werden kann unter Verwendung eines hierfür separaten technischen Verfahrens und/oder mikroelektronischer Vorrichtung. Dieses Verfahren für den Zugriff auf Teilfunktionen der internen Komponente verwendet bevorzugt ein magnetostatisches Prinzip, also z.B. die Bewegung eines zur internen Komponente gehörigen Hebels mit ferromagnetischer Komponente, ein induktives Prinzip, also die induktive Einwirkung auf die interne Komponente, ein schalltechnisches Prinzip, also die Einwirkung auf einen Schaltmechanismus in der internen Komponente durch Schallsignale, ein mechanisches Prinzip, also z.B. die Anregung von mechanischen Druck-, Sog-, Bewegungs-, oder Vibrationsdetektoren in der internen Komponente, bzw. die Anwendung anderer technisch bekannter physikalischer oder chemischer Prinzipien, um entsprechende Detektoren in der internen Komponente erreichen.

Eine weitere vorteilhafte Ausgestaltung des Verfahrens zum geschützten Betrieb besteht darin, dass die Aufhebung einer zuvor eingetretenen Sperre (siehe oben) mit Hilfe einer Authentifikation erfolgt, für die ein weiterer Schlüssel bei der Herstellung der Komponente in der Software und/oder der Hardware der Komponente implementiert wird, oder mit Hilfe eines im vorhergehenden Absatz genannten Verfahrens.

Eine weitere vorteilhafte Ausgestaltung des Verfahrens zum geschützten Betrieb besteht darin, dass die Verschlüsselung der zwischen externer und interner Komponente ausgetauschten Daten einschließlich des Autorisierungssignals in nicht zwingend periodischen Abständen wechselt. Es ist vorzugsweise vorgesehen, dass die Datenverschlüsselung und zugehörige Entschlüsselung vor Beginn der Autorisierung fest ist. Ferner ist vorzugsweise vorgesehen, dass sich die Datenverschlüsselung nach erfolgter Autorisierung in Abhängigkeit vom zuletzt verwendeten Autorisierungssignal in einer bei der Paarfertigung festgelegten Weise ändert.

Die Verschlüsselung der Datenübertragung erfolgt in dieser vorteilhaften Ausführung mit Hilfe eines Algorithmus zur Ver- und Entschlüsselung von Daten auf der Basis öffentlicher und privater Schlüssel. Jede externe und jede implantierte Komponente der Neuroprothese betreibt einen Schlüsselsatz bestehend aus einem privaten Schlüssel, der nur innerhalb der jeweiligen Komponente bekannt ist, und aus einem öffentlichen Schlüssel, der zusätzlich innerhalb der übrigen Komponenten bekannt ist. Bei der Herstellung der Komponenten werden diese jeweils mit einem öffentlichen und einem privaten Initialschlüssel sowie mit den öffentlichen Initialschlüsseln der anderen Komponenten ausgestattet. Jede Komponente ersetzt in zufälligen Zeitabständen in der Größenordnung einiger Sekunden den eigenen Schlüsselsatz automatisch. Jede Komponente gibt den öffentlichen Schlüssel an die anderen Komponenten verschlüsselt weiter, sobald dieser geändert wurde. Zum Zweck der Verschlüsselung der Daten verfügt jede Komponente über elektronische Leitungen, einen Speicher und einen Prozessor, die gemeinsam die Implementierung des Verschlüsselungsalgorithmus darstellen. Die Verschlüsselung der Daten erfolgt derart, dass der Verschlüsselungsalgorithmus aus den Daten, dem privaten Schlüssel der sendenden Komponente und aus dem öffentlichen Schlüssel der Komponente, an die die Daten gesendet werden sollen, neue Daten generiert. Diese Daten werden gesendet und von der empfangenden Komponente mittels des öffentlichen Schlüssels der sendenden Komponente und des privaten Schlüssels der empfangenden Komponente entschlüsselt. Der Entschlüsselungsalgorithmus ist derart gestaltet, dass empfangene Daten, die mit anderen als den oben vorgesehenen Schlüsseln verschlüsselt wurden, von der empfangenden Komponente ignoriert werden.

Eine weitere vorteilhafte Ausgestaltung des Verfahrens zum geschützten Betrieb besteht darin, dass die verschlüsselte Kommunikation nach dem Prinzip eines Verschlüsselungssystems mit einem öffentlichen und einem privaten Schüssel aufgebaut ist. Der Sender einer Nachricht (interne oder externe Komponente) verschlüsselt die Nachricht mit dem nur in der sendenden Komponente im bekannten privaten Schlüssel, der mikroelektronisch und/oder softwaretechnisch bei der Herstellung der Komponente eingestellt wird. Der Empfänger dieser Nachricht entschlüsselt diese anhand der Information, von welcher Komponente sie gesendet wurde und mit dem öffentlichen Schlüssel der sendenden Komponente, der mikroelektronisch und/oder softwaretechnisch bei der Herstellung der Komponente eingestellt wird. Es wird keine technische Vorrichtung zum Auslesen des eingestellten Schlüssels weder in der externen noch in der internen Komponente implementiert.

Eine weitere vorteilhafte Ausgestaltung des Verfahrens zum geschützten Betrieb besteht darin, dass eine Authentifikation zur Freischaltung der internen und der externen Komponenten verwendet wird. Dabei wird die Software und/oder Hardware der Komponenten mit Hilfe von Freischaltungsschlüsseln entschlüsselt bzw. initialisiert. Die Freischaltung ist nur dann möglich, wenn alle bei der Herstellung der Komponenten vordefinierten Komponenten vorhanden sind und miteinander kommunizieren. Es wird keine technische Vorrichtung zum unverschlüsselten Auslesen des eingestellten Freischaltungsschlüssels weder in der externen noch in der internen Komponente implementiert.

Eine weitere vorteilhafte Ausgestaltung des Verfahrens zum geschützten Betrieb besteht darin, dass um einen Nachbau der Software zu vermeiden, die Software-Komponenten der externen und/oder internen Komponenten bei der Herstellung in der verschlüsselten Form gespeichert werden. Zur Inbetriebnahme einer internen und/oder externen Komponente wird zuerst ein Entschlüsselungsprogramm gestartet, das die Freischaltungsschlüssel von allen anderen internen und/oder externen Komponenten durch den verschlüsselten Kanal bezieht und das Hauptprogramm der Komponente entschlüsselt. Wenn zumindest ein Schlüssel falsch empfangen wird, wird das Hauptprogramm falsch entschlüsselt und wird dadurch funktionsunfähig.

Eine weitere vorteilhafte Ausgestaltung des Verfahrens zum geschützten Betrieb besteht darin, dass die Authentifikation in einer vorbestimmten Reihenfolge abläuft, z.B. zuerst werden die Freischaltungsschlüssel aus internen Komponenten über den verschlüsselten Kanal ausgelesen und damit wird die Software der externen Komponenten entschlüsselt und deren Hardwarekomponente initialisiert. Alternativ erfolgt dies nach einem stochastischem Prinzip.

Eine weitere vorteilhafte Ausgestaltung des Verfahrens zum geschützten Betrieb besteht darin, dass die Freischaltungsschlüssel anhand der biometrischen Merkmale (z.B. Iris, Fingerabdruck, Stimme, genetischer Abdruck, Gehirnwellen, bioelektrische Eigenschaften der Gewebe) des Implantatträgers erstellt werden. Damit wird ein nichtautorisierter Betrieb der Neuroprothese in der Abwesenheit des Implantatträgers verhindert.

Eine weitere vorteilhafte Ausgestaltung des Verfahrens zum geschützten Betrieb besteht darin, dass eine Autorisierung jeder Komponente nach der Authentifikation und permanent bzw. wiederholt während des Betriebs erfolgt, und zur Detektion möglicher Angriffe dient. Die Autorisierung erfolgt mittels Autorisierungsschlüssel. Jede interne und externe Komponente beinhaltet einen eigenen Autorisierungsschlüssel, der entweder stochastisch und/oder anhand der Herstellerdaten und/oder biometrischen Daten des Implantatträgers erstellt wird und mikroelektronisch und/oder softwaretechnisch bei der Herstellung der Komponente eingestellt wird.

Eine weitere vorteilhafte Ausgestaltung des Verfahrens zum geschützten Betrieb besteht darin, dass zwischen zwei Arten des NIS-Betriebs unterschieden wird: Einstellungsbetrieb und autonomer Betrieb. Während des Einstellungsbetriebes findet eine Kommunikation zwischen dem Implantatträger und einem PC, oder zwischen dem Arzt und einem PC, oder zwischen einer anderen legitimierten Person und dem PC statt. Jeder Person wird ein unikales Passwort zugewiesen, das zusammen mit Autorisierungsschlüsseln der internen und/oder externen Komponenten die Berechtigungen zum Betrieb der Neuroprothese definieren. Die Passworte unterliegen der Geheimhaltungspflicht.

Eine weitere vorteilhafte Ausgestaltung des Verfahrens zum geschützten Betrieb besteht darin, dass der Einstellbetrieb der Neuroprothese nur nach einer Versetzung des Implantatträgers in einen für den autonomen Betrieb der Neuroprothese ungewöhnlichen psychischen und/oder physiologischen Zustand erfolgt, der mit vorhandenen elektromagnetischen bzw. elektrochemischen bzw. optischen bzw. thermischen bzw. mechanischen Sensoren identifiziert werden kann (z.B. Schlafzustand kann mittels Detektion von alpha- und beta-Wellen des Gehirns identifiziert werden, erhöhter pH-Wert kann mit einem pH-Sensor gemessen werden, erhöhte oder erniedrigte Körpertemperatur z.B. mittels Temperaturmessung, erhöhte Durchblutung der Haut z.B. mittels optischer Sensoren).

Eine weitere vorteilhafte Ausgestaltung des Verfahrens zum geschützten Betrieb besteht darin, dass im autonomen Betrieb eine Autorisierung nach einem Zustandsmuster erfolgt. Das Zustandsmuster wird durch Autorisierungsschlüssel und/oder interne Signale und/oder Zustände der internen und /oder externen Komponenten definiert (z.B. bei einer Neuroprothese werden die Stimulationssignale und/oder Zustände der spatialen und/oder temporalen Filter zur Beschreibung des Zustandsmusters benutzt). Die internen Signale und Zustände identifizieren eindeutig den Implantatträger und kommen in keiner anderen Neuroprothese in der Kombination des Zustandsmusters vor.

Eine weitere vorteilhafte Ausgestaltung des Verfahrens zum geschützten Betrieb besteht darin, dass die interne Komponente oder/und die externe Komponente sowohl im Falle unerwünschter Funktionen, wie einer Fehlfunktion der Neuroprothese, eines versuchten Betriebsmissbrauchs oder /und eines für den autorisierten Betrieb nicht vorgesehenen Zugriffsversuchs auf Daten oder Funktion der Neuroprothese, als auch im Falle bestimmter autorisierter Funktionen, wie z.B. bei der Abfrage von personenbezogenen Daten aus einer internen Komponente, gesonderte Signale auslöst. Dieses Alarmsignal im einen Fall bzw. Statussignal im anderen Fall wird in einer bevorzugten Ausgestaltung allein dem Implantatträger zur Kenntnis gebracht, z.B. durch Auslösung einer über Mechanorezeptoren wahrnehmbaren Empfindung.

## Patentansprüche

1. Neuroprothese, umfassend
eine für die Implantierung in den menschlichen Körper ausgelegte interne Komponente und eine externe Komponente, wobei zwischen der internen und der externen Komponente eine drahtlose Datenübertragung vorgesehen ist,
**dadurch gekennzeichnet,**
**dass** die Datenübertragung eines Teiles der Daten oder aller Daten nur dann erfolgt, wenn ein von der externen Komponente an die interne Komponente übermitteltes Autorisierungssignal geprüft und akzeptiert worden ist.

2. Neuroprothese nach Anspruch 1, **dadurch gekennzeichnet, dass** das von der internen Komponente akzeptierte Autorisierungssignal genau einer externen Komponente oder genau zwei externen Komponenten zugeordnet ist.

3. Neuroprothese nach Anspruch 1 oder 2, **dadurch gekennzeichnet, dass** die externe Komponente dazu ausgebildet ist, das von der internen Komponente akzeptierte Autorisierungssignal als Teil eines Datenstroms zu übermitteln.

4. Neuroprothese nach einem der vorangehenden Ansprüche, **dadurch gekennzeichnet, dass** die interne Komponente dazu ausgebildet ist, das Autorisierungssignals mittels eines programmierbaren Speichers zu überprüfen.

5. Neuroprothese nach einem der vorangehenden Ansprüche, **dadurch gekennzeichnet, dass** die interne Komponente dazu ausgebildet ist, das Autorisierungssignal mittels einer festen topologischen Halbleiterstruktur zu überprüfen.

6. Neuroprothese nach einem der vorangehenden Ansprüche, **dadurch gekennzeichnet, dass** für die Datenübertragung oder Funktion der internen Komponente mindestens zwei Betriebszustände möglich sind, die mittels unterschiedlicher Autorisierungssignale aktivierbar sind.

7. Neuroprothese nach einem der vorangehenden Ansprüche, **dadurch gekennzeichnet, dass** die interne Komponente dazu ausgebildet ist, im Falle einer Betriebsstörung mit einem zweiten Autorisierungsverfahren eine Abschaltung oder ein Notfallprogramm durchzuführen, wobei das zweite Autorisierungsverfahren vorzugsweise nach einem anderen Funktionsprinzip arbeitet als das erste Autorisierungsverfahren.

8. Neuroprothese nach einem der vorangehenden Ansprüche, **dadurch gekennzeichnet, dass** die interne Komponente so ausgebildet ist, dass sie im Betrieb mit einer weiteren implantierten Komponente Daten austauschen kann.

9. Neuroprothese nach einem der vorangehenden Ansprüche, **dadurch gekennzeichnet, dass** die Neuroprothese dazu ausgebildet ist, die Datenübertragung zu verschlüsseln, wobei vorzugsweise die interne Komponente einen programmierbaren oder mittels einer festen toplogischen Halbleiterstruktur definierten Schlüssel enthält.

10. Neuroprothese nach einem der vorangehenden Ansprüche, **dadurch gekennzeichnet, dass** die interne Komponente dazu ausgebildet ist, nach einer Anzahl von geprüften und nicht akzeptierten Autorisierungsversuchen die Annahme weiterer Autorisierungsversuche zeitweise oder permanent zu sperren und eine Aufhebung der Sperre nur mittels eines im normalen Betrieb nicht erforderlichen Autorisierungssignals zuzulassen.

11. Neuroprothese nach einem der vorangehenden Ansprüche, **dadurch gekennzeichnet, dass** die interne Komponente dazu ausgebildet ist, nach einer akzeptierten Autorisierung ein die Autorisierung anzeigendes Statussignal an die externe Komponente zu übermitteln.

12. Neuroprothese nach einem der vorangehenden Ansprüche, **dadurch gekennzeichnet, dass** das Autorisierungssignal ein bestimmtes Signalmuster in Form eines Frequenz- Zeit-Muster aufweist.

13. Neuroprothese nach einem der vorangehenden Ansprüche, **dadurch gekennzeichnet, dass** die interne Komponente dazu ausgebildet ist, das Autorisierungssignal unter Verwendung eines Schlüsselpaars zu überprüfen.

14. Neuroprothese nach einem der vorangehenden Ansprüche, **dadurch gekennzeichnet, dass** die interne Komponente und die externe Komponente Verschlüsselungshardware und/oder Verschlüsselungssoftware auf der Basis eines Prozessors und/oder programmierbarer Hardware und/oder von Speichern umfassen und dazu ausgebildet sind, den Betrieb nur nach erfolgreicher Authentifizierung und Autorisierung aller Komponenten zuzulassen.

15. Neuroprothese nach einem der vorangehenden Ansprüche, **dadurch gekennzeichnet, dass** die Neuroprothese dazu ausgebildet ist, das Autorisierungssignal durch ein elektromagnetisches Verfahren als frequenzmodulierte Impulsgruppe, durch ein opto-elektronisches Verfahren als amplitudenmodulierte Impulsgruppe von einer Laser-Diode als Sender zu einem Photosensor oder durch ein akustisches Verfahren durch die Modulation von Schall oder Körperschall mittels Vibrationsmustern an Mechanorezeptoren zu übermitteln.

16. Neuroprothese nach einem der vorangehenden Ansprüche, **dadurch gekennzeichnet, dass** die interne Komponente einen periodischen und einen nicht-periodischen Zeitgeber umfasst, der dazu ausgebildet ist, nicht-periodische Zeitcodes zu erzeugen, so dass zur erfolgreichen Autorisierung eine Kopie dieses nicht-periodischen Zeitgebers in der externen Komponente erforderlich ist, der mit dem nicht-periodischen Zeitcodes in der internen Komponente identische Zeitcodes erzeugen kann.

17. Neuroprothese nach einem der vorangehenden Ansprüche, **dadurch gekennzeichnet, dass** die interne Komponente dazu ausgebildet ist, im Falle der Ablehnung eines Autorisierungssignals entweder ein Ablehnungssignal abzugeben oder sich passiv zu verhalten.

18. Neuroprothese nach einem der vorangehenden Ansprüche, **dadurch gekennzeichnet, dass** zwischen der internen Komponente und der zugehörigen externen Komponente eine unverwechselbare Zuordnung in der Art eines komplementären Schlüssel-Schloss-Paars vorliegt, wobei die Zuordnung der internen Komponente und der zugehörigen externen Komponente mittels eines Zufallsprozess, mittels Lernalgorithmen oder unter Verwendung von persönlichen Körpermerkmalen erzeugbar ist.

19. Neuroprothese nach einem der vorangehenden Ansprüche, **dadurch gekennzeichnet, dass** die Neuroprothese dazu ausgebildet ist, den Zugriff auf Daten, die auf der internen Komponente oder der externen Komponente gespeichert sind, nur bei erfolgreicher Autorisierung zu ermöglichen.

20. Neuroprothese nach einem der vorangehenden Ansprüche, **dadurch gekennzeichnet, dass** die Neuroprothese dazu ausgebildet ist, ein in der internen Komponente legitimierten Autorisierungsversuch auch in der externen Komponente zu registrieren und die Datenübertragung sowie die Freigabe des Zuganges zu den in der externen Komponente gespeicherten Daten oder Funktionszuständen vorzunehmen.

21. Neuroprothese nach einem der vorangehenden Ansprüche, **dadurch gekennzeichnet, dass** die externe Komponente dazu ausgebildet ist, ein Autorisierungssignal zu erzeugen, das ein Signal-Muster ist, welches durch Amplituden-, Frequenz-, Zeit- oder Orts-Parameter festgelegt ist.

22. Neuroprothese nach einem der vorangehenden Ansprüche, **dadurch gekennzeichnet, dass** jede externe Komponente als Autorisierungssignal seine eindeutige Kennung als Autorisierungs-Schlüssel und jede interne Komponente ein anderes Autorisierungssignal als Autorisierungs-Schloss aufweist.

23. Neuroprothese nach einem der vorangehenden Ansprüche, **dadurch gekennzeichnet, dass** in einer internen Komponente gespeicherte personenbezogene Daten mit einer hierfür gesonderten Zugangssicherung geschützt sind und nur durch ein gesondertes Autorisierungssignal zugänglich sind.

24. Neuroprothese nach einem der vorangehenden Ansprüche, **dadurch gekennzeichnet, dass** die Neuroprothese dazu ausgebildet ist, die Datenübertragung zwischen der internen Komponente und die externen nach dem Prinzip eines Verschlüsselungssystems mit einem öffentlichen und einem privaten Schüssel durchzuführen, wobei die interne oder die externe Komponente dazu ausgebildet sind, beim Senden von Daten die Daten mit einem nur in der sendenden Komponente bekannten privaten Schlüssel zu verschlüsseln, der mikroelektronisch und/oder softwaretechnisch bei der Herstellung der Komponente einstellbar ist, und die empfangende Komponente ausgebildet ist, die verschlüsselten Daten mit dem öffentlichen Schlüssel der sendenden Komponente zu entschlüsseln, der mikroelektronisch und/oder softwaretechnisch bei der Herstellung der Komponente einstellbar ist.

25. Neuroprothese nach einem der vorangehenden Ansprüche, **dadurch gekennzeichnet, dass** die Neuroprothese dazu ausgebildet ist, durch das Zusammenwirken von Speicher, Prozessor und/oder programmierbarer Mikroelektronik der internen Komponente ein elektronisches Schloss zu erzeugen, das nur von dem entsprechenden Autorisierungssignal als Schlüssel korrekt durchlaufen werden kann.

26. Neuroprothese nach Anspruch 14, **dadurch gekennzeichnet, dass** die interne Komponente dazu ausgebildet ist, beim korrekten Durchlaufen des elektronischen Schlosses in der internen Komponente durch das entsprechende Autorisierungssignal ein zeitliches Signalmuster zu erzeugen, das auf eine logische Gatterstruktur geleitet und als erfolgreiche Autorisierung interpretiert werden kann.

## Claims

1. Neuroprosthesis, comprising
a component designed to be implanted in the human body and an external component, a wireless data transmission being provided between the internal and the external component,
**characterised in that**
the data transmission of some of the data or all of the data does not take place unless an authorisation signal transmitted by the external component to the internal component has been checked and accepted.

2. Neuroprosthesis as claimed in claim 1, **characterised in that** the authorisation signal accepted by the internal component is assigned specifically to an external component or specifically to two external components.

3. Neuroprosthesis as claimed in claim 1 or 2, **characterised in that** the external component is configured to transmit the authorisation signal accepted by the internal component as part of a data stream.

4. Neuroprosthesis as claimed in one of the preceding claims, **characterised in that** the internal component is configured to check the authorisation signal by means of a programmable memory.

5. Neuroprosthesis as claimed in one of the preceding claims, **characterised in that** the internal component is configured to check the authorisation signal by means of a fixed topological semiconductor structure.

6. Neuroprosthesis as claimed in one of the preceding claims, **characterised in that** at least two operating modes are possible for the data transmission or operation of the internal component, which can be activated by means of different authorisation signals.

7. Neuroprosthesis as claimed in one of the preceding claims, **characterised in that** the internal component is configured to switch off or run an emergency programme with a second authorisation procedure in the event of an operating fault, and the second authorisation procedure preferably operates on the basis of a different operating principle from the first authorisation procedure.

8. Neuroprosthesis as claimed in one of the preceding claims, **characterised in that** the internal component is configured so that it can exchange data with another implanted component during operation.

9. Neuroprosthesis as claimed in one of the preceding claims, **characterised in that** the neuroprosthesis is configured to encrypt the data transmission, and the internal component preferably contains a key which is programmable or is defined by means of a fixed topological semiconductor structure.

10. Neuroprosthesis as claimed in one of the preceding claims, **characterised in that**, if a number of authorisation attempts have been checked and not accepted, the internal component is configured to block the acceptance of further authorisation attempts for a period of time or permanently and not allow the block to be lifted except by an authorisation signal other than the one needed for normal operation.

11. Neuroprosthesis as claimed in one of the preceding claims,
**characterised in that**, having accepted an authorisation, the internal component is configured to transmit a status signal to the external component indicating the acceptance.

12. Neuroprosthesis as claimed in one of the preceding claims, **characterised in that** the authorisation signal has a specific signal pattern in the form of a frequency-time pattern.

13. Neuroprosthesis as claimed in one of the preceding claims, **characterised in that** the internal component is configured to check the authorisation signal using a pair of keys.

14. Neuroprosthesis as claimed in one of the preceding claims, **characterised in that** the internal component and the external component have encryption hardware and/or encryption software based on a processor and/or programmable hardware and/or from memories, and are configured to permit operation only when all the components have been successfully authenticated and authorised.

15. Neuroprosthesis as claimed in one of the preceding claims, **characterised in that** the neuroprosthesis is configured to transmit the authorisation signal by means of an electromagnetic method as a frequency-modulated pulse group, by means of an opto-electronic method as an amplitude-modulated pulse group from a laser diode as the transmitter to a photo-sensor, or by an acoustic method by modulating sound or structure-borne noise by means of vibration patterns to mechanical receptors.

16. Neuroprosthesis as claimed in one of the preceding claims, **characterised in that** the internal component has a periodic and a non-periodic time transmitter, which, in order to complete authorisation successfully, is configured to generate non-periodic time codes so that a copy of this non-periodic time transmitter is needed in the external component, which is able to generate time codes identical to the non-periodic time codes in the internal component.

17. Neuroprosthesis as claimed in one of the preceding claims, **characterised in that**, if an authorisation signal is rejected, the internal component is configured either to emit a rejection signal or to assume a passive behaviour.

18. Neuroprosthesis as claimed in one of the preceding claims, **characterised in that** a non-changeable allocation exists between the internal component and the co-operating external component in the form of a complementary key-lock pair, and the allocation of the internal component and the co-operating external component is generated by means of a random process, by means of learning algorithms or using personal physical characteristics.

19. Neuroprosthesis as claimed in one of the preceding claims, **characterised in that** the neuroprosthesis is configured so that it does not permit access to data stored on the internal component or external component unless authorisation is successful.

20. Neuroprosthesis as claimed in one of the preceding claims, **characterised in that** the neuroprosthesis is configured to log an authorisation attempt that has been legitimised in the external component as well and assume responsibility for the data transmission and release access to the data or functional states stored in the external component.

21. Neuroprosthesis as claimed in one of the preceding claims, **characterised in that** the external component is configured to generate an authorisation signal comprising a signal pattern which is fixed on the basis of amplitude, frequency, time or location parameters.

22. Neuroprosthesis as claimed in one of the preceding claims, **characterised in that**, as an authorisation signal, every external component has its unique code comprising an authorisation key and every internal component has a different authorisation signal comprising an authorisation lock.

23. Neuroprosthesis as claimed in one of the preceding claims, **characterised in that** personal data stored in an internal component is protected by a separate access key specifically provided for it and is not accessible except by a separate authorisation signal.

24. Neuroprosthesis as claimed in one of the preceding claims, **characterised in that** the neuroprosthesis is configured to run the data transmission between the internal and the external component based on the principle of an encryption system with a public and a private key, and when transmitting data, the internal or the external component is configured to encrypt the data with a private key known only in the transmitting component, which can be set micro-electronically and/or by means of software when the component is manufactured, and the receiving component is configured to decrypt the encrypted data with the public key of the transmitting component, which can be set micro-electronically and/or by software when the component is manufactured.

25. Neuroprosthesis as claimed in one of the preceding claims, **characterised in that**, in co-operation with a memory, processor and/or programmable micro-electronic system of the internal component, the neuroprosthesis is configured to generate an electronic lock which can only be run correctly by the corresponding authorisation signal in the form of a key.

26. Neuroprosthesis as claimed in claim 14, **characterised in that**, when the electronic lock in the internal component is run correctly, the internal component is configured to generate a time-based signal pattern by means of the appropriate authorisation signal, which can be directed to a logical gate structure and interpreted as a successful authorisation.

## Revendications

1. Neuroprothèse comprenant
un composant interne réalisé pour l'implantation dans le corps humain et un composant externe, un transfert de données sans fil étant prévu entre le composant interne et le composant externe,
**caractérisée en ce**
**que** le transfert de données d'une partie des données ou de toutes les données ne se fait que lorsqu'un signal d'autorisation transmis au composant interne par le composant externe a été vérifié et accepté.

2. Neuroprothèse selon la revendication 1, **caractérisée en ce que** le signal d'autorisation accepté par le composant interne est associé exactement à un composant externe ou exactement à deux composants externes.

3. Neuroprothèse selon la revendication 1 ou 2, **caractérisée en ce que** le composant externe est réalisé pour transmettre le signal d'autorisation accepté par le composant interne comme une partie d'un flux de données.

4. Neuroprothèse selon l'une quelconque des revendications précédentes, **caractérisée en ce que** le composant interne est réalisé pour vérifier le signal d'autorisation au moyen d'une mémoire programmable.

5. Neuroprothèse selon l'une quelconque des revendications précédentes, **caractérisée en ce que** le composant interne est réalisé pour vérifier le signal d'autorisation au moyen d'une structure topologique fixe à semi-conducteurs.

6. Neuroprothèse selon l'une quelconque des revendications précédentes, **caractérisée en ce que**, pour le transfert de données ou le fonctionnement du composant interne, au moins deux états de fonctionnement sont possibles, qui peuvent être activés au moyen de différents signaux d'autorisation.

7. Neuroprothèse selon l'une quelconque des revendications précédentes, **caractérisée en ce que** le composant interne est réalisé pour exécuter une déconnexion ou un programme d'urgence avec un deuxième procédé d'autorisation en cas de dysfonctionnement, le deuxième procédé d'autorisation fonctionnant, de préférence, selon un autre principe de fonctionnement que celui du premier procédé d'autorisation.

8. Neuroprothèse selon l'une quelconque des revendications précédentes, **caractérisée en ce que** le composant interne est réalisé de sorte qu'il peut échanger des données avec un autre composant implanté pendant son fonctionnement.

9. Neuroprothèse selon l'une quelconque des revendications précédentes, **caractérisée en ce que** la neuroprothèse est réalisée pour coder le transfert de données, le composant interne contenant, de préférence, une clé programmable ou définie au moyen d'une structure topologique fixe à semi-conducteurs.

10. Neuroprothèse selon l'une quelconque des revendications précédentes, **caractérisée en ce que**, après un certain nombre d'essais d'autorisation vérifiés et non acceptés, le composant interne est réalisé pour bloquer temporairement ou en permanence l'acceptation d'autres essais d'autorisation et pour autoriser la levée du blocage uniquement au moyen d'un signal d'autorisation non nécessaire en fonctionnement normal.

11. Neuroprothèse selon l'une quelconque des revendications précédentes, **caractérisée en ce que** le composant interne est réalisé pour transmettre au composant externe un signal d'état indiquant l'autorisation après une autorisation acceptée.

12. Neuroprothèse selon l'une quelconque des revendications précédentes, **caractérisée en ce que** le signal d'autorisation comporte un modèle de signal déterminé sous forme de modèle fréquence-temps.

13. Neuroprothèse selon l'une quelconque des revendications précédentes, **caractérisée en ce que** le composant interne est réalisé pour vérifier le signal d'autorisation à l'aide d'une paire de clés.

14. Neuroprothèse selon l'une quelconque des revendications précédentes, **caractérisée en ce que** le composant interne et le composant externe comportent un matériel de codage et/ou un logiciel de codage sur la base d'un processeur et/ou d'un matériel programmable et/ou de mémoires et sont réalisés pour autoriser le fonctionnement uniquement après une authentification et une autorisation de tous les composants réussies.

15. Neuroprothèse selon l'une quelconque des revendications précédentes, **caractérisée en ce que** la neuroprothèse est réalisée pour transmettre le signal d'autorisation à des mécanorécepteurs à l'aide d'un procédé électromagnétique sous forme de groupe d'impulsions à modulation de fréquence, à l'aide d'un procédé optoélectronique sous forme de groupe d'impulsions à modulation d'amplitude à partir d'une diode laser comme émetteur vers un photocapteur ou à l'aide d'un procédé acoustique par la modulation du son ou du bruit du corps au moyen de modèles vibratoires.

16. Neuroprothèse selon l'une quelconque des revendications précédentes, **caractérisée en ce que** le composant interne comprend un temporisateur périodique et un temporisateur non périodique qui est réalisé pour générer des codes de temps non périodiques de sorte que, pour la réussite de l'autorisation, une copie de ce temporisateur non périodique est nécessaire dans le composant externe, qui peut générer des codes de temps identiques dans le composant interne avec les codes de temps non périodiques.

17. Neuroprothèse selon l'une quelconque des revendications précédentes, **caractérisée en ce que** le composant interne est réalisé pour émettre un signal de refus ou pour se comporter passivement en cas de refus d'un signal d'autorisation.

18. Neuroprothèse selon l'une quelconque des revendications précédentes, **caractérisée en ce qu'**une association irréversible à la manière d'une paire complémentaire clé-verrou est présente entre le composant interne et le composant externe associé, l'association du composant interne et du composant externe associé pouvant être générée au moyen d'un processus aléatoire, au moyen d'algorithmes ou en utilisant des caractéristiques corporelles personnelles.

19. Neuroprothèse selon l'une quelconque des revendications précédentes, **caractérisée en ce que** la neuroprothèse est réalisée pour permettre l'accès à des données qui sont mémorisées dans le composant interne ou dans le composant externe uniquement en cas d'autorisation réussie.

20. Neuroprothèse selon l'une quelconque des revendications précédentes, **caractérisée en ce que** la neuroprothèse est réalisée pour enregistrer un essai d'autorisation légitimé dans le composant interne également dans le composant externe et pour réaliser le transfert de données ainsi que l'autorisation d'accès aux données ou aux états de fonctionnement mémorisés dans le composant externe.

21. Neuroprothèse selon l'une quelconque des revendications précédentes, **caractérisée en ce que** le composant externe est réalisé pour générer un signal d'autorisation qui est un modèle de signal qui est défini par des paramètres d'amplitude, de fréquence, de temps ou de lieu.

22. Neuroprothèse selon l'une quelconque des revendications précédentes, **caractérisée en ce que** chaque composant externe comporte comme signal d'autorisation son identification univoque comme clé d'autorisation et chaque composant interne comporte un autre signal d'autorisation comme verrou d'autorisation.

23. Neuroprothèse selon l'une quelconque des revendications précédentes, **caractérisée en ce que** les données personnelles mémorisées dans un composant interne sont protégées par une sécurité d'accès spécifique pour cela et sont accessibles seulement à l'aide d'un signal d'autorisation spécifique.

24. Neuroprothèse selon l'une quelconque des revendications précédentes, **caractérisée en ce que** la neuroprothèse est réalisée pour effectuer le transfert de données entre le composant interne et le composant externe suivant le principe d'un système de codage avec une clé publique et un clé privée, le composant interne ou le composant externe étant réalisé, lors de l'envoi de données, pour coder les données avec une clé privée connue uniquement dans le composant émetteur qui peut être réglée par un moyen microélectronique et/ou par une technique de logiciel lors de la fabrication du composant et le composant récepteur est réalisé pour décoder les données codées avec la clé publique du composant émetteur qui peut être réglée par un moyen microélectronique et/ou par une technique de logiciel lors de la fabrication du composant.

25. Neuroprothèse selon l'une quelconque des revendications précédentes, **caractérisée en ce que** la neuroprothèse est réalisée pour générer un verrou électronique par l'interaction de la mémoire, du processeur et/ou de la microélectronique programmable du composant interne, lequel verrou ne peut être pénétré correctement que par le signal d'autorisation correspondant sous forme de clé.

26. Neuroprothèse selon la revendication 14, **caractérisée en ce que**, en cas de pénétration correcte du verrou électronique dans le composant interne par le signal d'autorisation correspondant, le composant interne est réalisé pour générer un modèle de signal temporel qui peut être dirigé vers une structure de porte logique et interprété comme une autorisation réussie.
